Europäisches Patentamt

European Patent Office

Office européen des brevets

(19)

(11) Veröffentlichungsnummer: **0 170 014**
**B1**

(12) **EUROPÄISCHE PATENTSCHRIFT**

(45) Veröffentlichungstag der Patentschrift:
04.01.89

(51) Int. Cl.⁴: **C 07 C 67/03, C 07 C 67/08**

(21) Anmeldenummer: **85107286.8**

(22) Anmeldetag: **13.06.85**

(54) **Verfahren zur Herstellung von Wachsestern.**

(30) Priorität: **28.07.84 DE 3427884**

(43) Veröffentlichungstag der Anmeldung:
**05.02.86 Patentblatt 86/6**

(45) Bekanntmachung des Hinweises auf die Patenterteilung:
**04.01.89 Patentblatt 89/1**

(84) Benannte Vertragsstaaten:
**BE DE FR GB IT NL**

(56) Entgegenhaltungen:
**DE-A-2 432 216**
**DE-C-1 274 105**
**GB-A-1 437 039**

**SOVIET INVENTIONS ILLUSTRATED, DERWENT PUBLICATIONS LTD., Section Chemical, Woche 84, 04. Juli 1984, Abstract Nr. 21, Derwent Publications Ltd., London, GB; & SU - A - 1 038 336 (CHEM IND RES DES IN) 30.08.1983**
**CHEMICAL ABSTRACTS, Band 89, Nr. 12, 18. September 1978, Seite 78, Spalten 1, 2, Abstract Nr. 91420p, Columbus, Ohio, US; I. SZABO et al.: "Investigations on the new preparation possibilities of Span 80 and Tween 80", & Proc. Conf. Appl. Chem., Unit Oper. Processes 3rd 1977, 487-491**

(73) Patentinhaber: **HÜLS AKTIENGESELLSCHAFT, - RSP Patente / PB 15 - Postfach 13 20, D-4370 Marl 1 (DE)**

(72) Erfinder: **Kühnle, Adolf, Dr., Lipperweg 195, D-4370 Marl (DE)**
Erfinder: **Kehr, Helmut, Dr., Gustav- Sack- Strasse 8, D-4235 Schermbeck (DE)**

EP 0 170 014 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Gegenstand der Erfindung ist ein verbessertes Veresterungsverfahren für die Oxidationsprodukte aus synthetischen Hartparaffinen.

Zwar sind Veresterungen weitgehend erforscht. So kann man die unkatalysiert langsam verlaufende Veresterung niederer Fettsäuren durch Mineral- oder Lewis-Säuren beschleunigen, wobei das reaktionsschwache mesomeriestabilisierte Carboxylat-Anion in ein reaktionsfähiges Kation übergeht, an welches sich das nucleophile Sauerstoffatom des Alkohols anlagert; diese Technik wird aber schon bei höheren Fettsäuren weniger wirksam, teils wegen deren geringerer Beweglichkeit, teils wegen der reaktionsschwächer werdenden Carboxylfunktion.

Umgekehrt läßt sich, im Gegensatz zu der Veresterung niederer Fettsäuren, die mit Alkalien ein hinderliches stabiles Carboxylat-Anion bilden, die Veresterung höherer Fettsäuren mit Glykolen durch Alkalihydroxid beschleunigen (Pardun, Seifen-Öle-Fett-Wachse 106, 65, 27 (1981). Schließlich war auch bekannt, daß verschiedene Metallpulver, -oxide und -salze die Veresterungen von Fettsäuren katalysieren können.

Diese Verfahren sind jedoch auf synthetische Wachse nicht zu übertragen, wie die DE-AS-2 201 862 und DE-AS-2 432 215 lehren.

Die DE-AS-2 201 862 beschreibt die Veresterung von Polyethylenoxidaten mit ein- oder mehwertigen Alkoholen in Gegenwart von Schwefelsäure. Nach den Beispielen werden jedoch bei 115°C maximal die Hälfte der durch die Säurezahl erfaßten Wachssäuren verestert; bei höheren Temperaturen sind unerwünschte Verfärbungen zu befürchten. Die beanspruchte vollständige Veresterung ist demnach nicht leicht zu erreichen, und so spricht die Schrift von geringer Veresterungsbereitschaft und langer Reaktionszeit. Nachteilig ist auch das Erfordernis der nachfolgenden Säureneutralisation.

Aus SU-A-1 038 336 (Soviet Inventions Illustrated, Derwent Publications Ltd., Section Chemical, Woche 84, 04. Juli 1984, Abstract Nr. 21, Derwent Publications Ltd., London, GB) war zwar bekannt, ein synthetisches modifiziertes Wachs durch eine Veresterung mittels Zinkoxid als Katalysator herzustellen. Hierbei handelt es sich aber nicht um die erfindungsgemäße Umsetzung synthetischer Hartparaffin-Oxidate mit Alkoholen zu reinen Wachsen, sondern um die Herstellung modifizierter Wachse aus Fettsäuren und allgemein hydroxylgruppenhaltigen Aliphaten.

Wie sich gemäß Beispiel 1 aus dem der Reaktion nachgeschalteten Neutralisationsschritt errechnen läßt, gelingt die Veresterung nur zu 29 %. Die verbliebenen Säuregruppen, nämlich 71 %, werden mit 0,5 g Zinkoxid und immerhin 4,5 g Natriumhydroxid, bezogen auf 49,75 g Stearinsäure, neutralisiert. Ausgangsstoffe sind Fettsäuren natürlicher Herkunft mit endständiger Carboxylgruppe einerseits und ein hydroxylgruppenhaltiges Fettsäureamid andererseits. Das Erzeugnis ist ein Gemisch, welches neben nicht umgesetzten Ausgangsstoffen die Reaktionsprodukte Zinkstearat, Natriumstearat, Stearinsäure-Fettsäuremonoethanolamid-Kondensat sowie weniger definierbare Folgeprodukte enthalten muß, die während der verschiedenen Reaktionsphasen und Aufheizperioden insbesondere bei der Behandlung mit 50 %-iger Natronlauge bei 100°C durch Ester- und Amidspaltung entstehen.

Es war nicht zu erwarten, daß Zinkverbindungen beim Wechsel der Einsatzstoffe viel wirksamer werden könnten. Der Fachmann mußte annehmen, daß die Fettsäuren mit ihren exponierten Carboxylgruppen leichter und vollständiger umzusetzen sein würden als die Oxidate mit ihren ungeregelt angeordneten Carboxylgruppen, und er mußte erwarten, daß Zinkverbindungen zu keiner auch nur annähernd vollständigen Umsetzung taugen, so daß man, falls man nicht zur nachfolgenden gründlichen Neutralisation gezwungen sein wollte, besser gleich einen anderen Katalysator suchte.

Es war also insgesamt nicht erkennbar, wie man die gestellte technische Aufgabe, nämlich eine weitgehende bis vollständige Veresterung der Oxidate unter schonenden Bedingungen, einfach lösen könnte.

Diese Aufgabe wird in der aus den Patentansprüchen ersichtlichen Weise gelöst.

Die Veresterung gelingt bei 140 bis 220°C, bevorzugt 150 bis 200°C, insbesondere 160 bis 190°C. Als Zinkverbindungen eignen sich einerseits Zinkoxid, Zinkhydroxid sowie Zinkmineralsäuresalze, andererseits Zinksalze von Carbonsäuren wie Zinkstearat.

Man setzt die Zinkverbindung in Mengen von 0,01 bis 3,0, bevorzugt 0,05 bis 2,0, insbesondere 0,1 bis 1,0 Gewichtsprozent, bezogen auf die Einsatzmenge Hartparaffin-Oxidat zu.

Geeignete Hartparaffin-Oxidate sind Fischer-Tropsch -Oxidate mit einem Tropfpunkt von 90 bis 110°C und Polyethylenwachsoxidate mit einem Tropfpunkt von 95 bis 140°C. Bevorzugt verwendet man solche, welche aus geschmolzenen Hartparaffinen durch Einblasen von Luft bei 120 bis 250°C, insbesondere 130 bis 180°C, entstehen, bevorzugt in Gegenwart eines Katalysators. Als Katalysator dient insbesondere ein Zinksalz, vor allem eine Zinkseife wie Zinkstearat, oder ein sogenannter Impfstoff, welcher seinerseits ein Hartparaffin-Oxidat ist. Man oxidiert die Hartparaffine bis zu einer Säurezahl von 5 bis 80, bevorzugt 10 bis 60; die Verseifungszahlen solcher Produkte liegen zwischen 8 und 150, bevorzugt 16 und 120. Man kann für die Veresterung auch teilverseifte Oxidate verwenden.

Die Teilverseifung führt man aus, indem man, entweder kontinuierlich während der Oxidation oder nach Beendigung der Oxidation, in der Schmelze die Säuregruppen des Wachsoxidats mit Basen wie Natriumhydroxid, Kaliumhydroxid, Lithiumhydroxid, Calciumhydroxid oder Zinkhydroxid bzw. mit Mischungen dieser Basen teilweise neutralisiert.

Als Hartparaffine, welche man der erforderlichen Oxidation unterworfen hat, eignen sich synthetische Hartparaffine, hergestellt nach dem Fischer-Tropsch-Verfahren, mit einem Tropfpunkt (DGF-M-III 3) zwischen 100 und 120° C und einem Molekulargewicht (dampfdruckosmometrisch bestimmt) von 600 bis 800 sowie Polyethylenhartparaffine. Bevorzugt verwendet man solche, wie sie bei der bekannten Ethylenpolymerisation nach Ziegler mit einem Molekulargewicht (dampfdruckosmometrisch bestimmt) von 1 000 bis 4 000, bevorzugt von 1 000 bis 3 000, gezielt oder als Nebenprodukt anfallen; solche Hartparaffine besitzen einen Tropfpunkt zwischen 100 und 140° C und eine Dichte zwischen 0,91 und 0,97 $g/cm^3$.

Geeignete einwertige Alkohole sind aliphatische Alkohole mit Kettenlängen von 4 bis 20 Kohlenstoffatomen und endständiger Hydroxylgruppe, bevorzugt mit Kettenlängen von 6 bis 12 Kohlenstoffatomen.

Als zwei- und höherwertige Alkohole, die man bevorzugt verwendet eignen sich Alkohole mit 2 bis 5 Kohlenstoffatomen und mindestens einer endständigen Hydroxylgruppe, insbesondere 1,2-Ethandiol, 1,2-Propandiol, 1,3-Propandiol, 1,3 -Butandiol, 1,4 -Butandion, Glycerin und Pentaerythrit.

Der Alkoholzusatz erfolgt stöchiometrisch zur halben bis doppelten Säurezahl, bevorzugt in Mengen von 0 bis 50 % über der zur Säurezahl stöchiometrischen Menge. Gegebenenfalls wird überschüssiger Alkohol nach der Veresterung durch Anlegen von Vakuum aus der Reaktionsmischung entfernt.

Die Veresterung wird in einem Rührgefäß in homogener Mischung unter Normaldruck oder unter reduziertem Druck bei einer Temperatur zwischen 140 bis 220° C durchgeführt. Das bei der Reaktion entstehende Wasser wird als Dampf über einen Destillationsaufsatz abgeführt und an einem Kühler kondensiert. Für Veresterungen unter reduziertem Druck ist der Destillationsaufsatz bzw. der Kühler nicht zwingend erforderlich.

Vorzugsweise verestert man die gegebenenfalls teilverseiften Hartparaffin-Oxidate vollständig, mindestens jedoch zu 70 % der durch die Säurezahl erfaßten Wachssäuren, wobei gleichzeitig die Säurezahl des gebildeten Wachsesters unter 10 mg KOH/g sinkt.

Ein Vorteil der neuen Veresterungsweise ist, daß man sicher oberhalb 160° C ohne Verfärbung des Erzeugnisses arbeiten kann, so daß die Umsetzung verhältnismäßig schnell abläuft. So gelingt es leicht, die Säurezahl eines oxidierten Polyethylenwachses von anfänglich 65 bei der Veresterung mit Ethylenglykol innerhalb 5 Stunden auf unter 10 zu senken; die halbe Säurezahl ist schon nach weniger als 2 Stunden erreicht.

Ein weiterer Vorteil ist, daß der Katalysator keiner Neutralisation bedarf, sondern im Gegenteil für die üblichen Anwendungen im Wachsester verbleiben kann.

Ein besonderer und überraschender Vorteil zeigt sich dann, wenn man für die Veresterung ein solches Hartparaffin-Oxidat einsetzt, bei dessen Herstellung eine Zinkseife als Katalysator gedient hat. Man kommt dann bei beiden Verfahrensstufen mit dem gleichen Katalysator, z. B. Zinkstearat, aus, und hat hierbei den Nutzen aus einer in der Chemie überaus seltenen Möglichkeit.

Die erfindungsgemäß erhaltenen Wachsester zeichnen sich gegenüber den nichtveresterten Hartparaffin-Oxidaten durch eine höhere Polarität aus. Aus diesem Grund sind sie, in Verbindung mit polaren Lösemittelsystemen, besonders zur Herstellung feinteiliger Wachsdispersionen geeignet. Aufgrund ihrer guten Verträglichkeit mit Lack- und Druckfarbenbindemitteln können sie Druckfarben und Lacken zur Erhöhung der Scheuerfestigkeit bzw. Kratzfestigkeit zugesetzt werden, ohne daß der Glanz wesentlich vermindert wird. Eine bevorzugte Anwendungsform betrifft den Einsatz als Gleitmittel bei der Verarbeitung thermoplastischer Kunststoffe, vor allem PVC, und hier insbesondere das Walzen und Kalandern bei der Herstellung von Hartfolien. Diese Verwendung ist Gegenstand einer gesonderten Patentanmeldung vom gleichen Tage.

## Beispiel 1

In einem 1 000-ml-Dreihalskolben, der mit einem Claisenaufsatz mit Liebig-Kühler versehen ist, rührt man 500 g eines Polyethylenwachsoxidates (Tropfpunkt 103° C, Penetration 4 mm · $10^{-1}$, Säurezahl 25 mg KOH/g, Verseifungszahl 49 mg KOH/g, Molekulargewicht (Mn) 1 900), 21 g 1,2 -Ethandiol und 2,5 g Zinkstearat bei einer konstanten Temperatur von 180° C. Man fängt das sich am Kühler abscheidende Reaktionswasser in einer Vorlage auf. Man kontrolliert das Fortschreiten der Reaktion anhand von Säurezahlbestimmungen. Die Reaktion ist nach 3 Stunden beendet. Nach Entfernen des nichtumgesetzten Ethandiols im Wasserstrahlvakuum erhält man ein fast weißes Wachs mit einer Säurezahl von 4 mg KOH/g. Das Produkt besitzt einen Tropfpunkt von 102° C und eine Penetration von 4 mm · $10^{-1}$.

## Vergleichsbeispiel 1

Man verfährt wie in Beispiel 1, nur daß man kein Zinkstearat zusetzt. In diesem Fall liegt die Säurezahl nach 3 Stunden noch bei 13 mg KOH/g. Obwohl man die Reaktion weitere 3 Stunden fortsetzt, sinkt die Säurezahl nicht unter 7 mg KOH/g.

**Beispiel 2**

Man verfährt wie in Beispiel 1, verwendet aber ein Polyethylenwachsoxidat (Tropfpunkt 107°C, Penetration 2 mm · 10⁻¹, Säurezahl 24 mg KOH/g, Verseifungszahl 43 mg KOH/g, Molekulargewicht (Mn) 1 300), welches - als Oxidationskatalysator - bereits 0,5 Gewichtsprozent Zinkstearat enthält, und man gibt zusätzlich kein Zinkstearat zu. Bereits nach 2 Stunden ist die Säurezahl auf 6 mg KOH/g gefallen, nach 3,5 Stunden ist die Reaktion beendet. Es wird ein fast weißes Wachs mit einer Säurezahl von 3 mg KOH/g erhalten.

**Beispiel 3**

In einem 2 000-ml-Dreihalskolben, der mit einem Claisenaufsatz mit Liebig-Kühler versehen ist, rührt man 800 g eines Polyethylenwachsoxidates (Tropfpunkt 104°C, Penetration 4 mm · 10⁻¹ Säurezahl 63 mg KOH/g, Verseifungszahl 112 mg KOH/g), welches bereits 0,5 Gewichtsprozent Zinkstearat - als Oxidationskatalysator - enthält, mit 83 g 1,2-Ethandiol bei einer konstanten Temperatur von 180°C. Das Fortschreiten der Reaktion wird anhand von Säurezahlbestimmungen kontrolliert. Bereits nach 2 Stunden ist die Säurezahl um 41 Einheiten auf 22 mg KOH/g abgefallen. Nach 5 Stunden beendet man die Reaktion bei einer Säurezahl von 7 mg KOH/g und entfernt nichtumgesetztes Ethandiol im Wasserstrahlvakuum. Man erhält ein fast weißes Wachs mit einem Tropfpunkt von 103°C und einer Penetration von 4 mm · 10⁻¹.

**Beispiel 4**

Man verfährt wie in Beispiel 3, setzt aber 1 000 g Polyethylenwachsoxidat mit 101 g 1,4-Butandiol um. Bereits nach 2 Stunden ist die Säurezahl um über die Hälfte auf 28 g KOH/g abgesunken. Nach 6 Stunden wird die Reaktion bei einer Säurezahl von 8 mg KOH/g beendet.

**Beispiel 5**

Man verfährt wie in Beispiel 3, setzt aber 800 g Polyethylenwachsoxidat mit 85 g Glycerin um. Nach 2 Stunden ist die Säurezahl um über die Hälfte auf 30 mg KOH/g abgesunken. Nach 7 Stunden wird die Reaktion bei einer Säurezahl von 9 mg KOH/g beendet.

**Beispiel 6**

Man verfährt wie in Beispiel 3, setzt aber 800 g Polyethylenwachsoxidat mit 123 g Pentaerythrit um. Nach 7 Stunden wird die Reaktion bei einer Säurezahl von 9 mg KOH/g beendet.

**Beispiel 7**

Man verestert 1 000 g eines mit Lithiumhydroxid und Calciumhydroxid teilverseiften (15 Säureeinheiten) Polyethylenwachsoxidates (Tropfpunkt 104°C, Penetration 1 mm · 10⁻¹, Säurezahl 25 mg KOH/g, Verseifungszahl 51 mg KOH/g, Molekulargewicht (Mn) 1 400), welches bereits als Oxidationskatalysator - 0,5 Gewichtsprozent Zinkstearat enthält, mit 28 g 1,2-Ethandiol bei einer Temperatur von 180°C. Nach 3 Stunden wird die Reaktion bei einer Säurezahl vom 6 mg KOH/g beendet.

**Beispiel 8**

Man verestert 1 000 g eines Fischer-Tropsch-Oxidates (Tropfpunkt 101°C, Penetration 5 mm · 10⁻¹, Säurezahl 30 mg KOH/g, Verseifungszahl 55 mg KOH/g, Molekulargewicht (Mn) 600), bei 180°C in einem 2 000-ml-Dreihalskolben - analog Beispiel 1 - mit 53 g 1,2-Ethandiol unter Zusatz von 5 g Zinkstearat. Nach 1 Stunde sind bereits 80 % der Säuregruppen verestert, nach einer weiteren Stunde ist die Säurezahl auf 3 abgesunken. Es wird ein fast weißes Produkt mit einem Tropfpunkt von 101°C und einer Penetration von 5 mm · 10⁻¹ erhalten.

**Vergleichsbeispiel 2**

Man verfährt wie in Beispiel 8, verwendet aber kein Zinkstearat. In diesem Fall liegt die Säurezahl nach einer Stunde noch bei 22 mg KOH/g. Nach 7 Stunden wird die Reaktion bei einer Säurezahl von 12 mg KOH/g abgebrochen.

**Beispiel 9**

Man verfährt wie in Beispiel 8, setzt aber anstelle von Zinkstearat 1 g Zinkchlorid ein. Nach 2 Stunden ist eine Säurezahl von 8 mg KOH/g erreicht. Nach 2 weiteren Stunden wird ein Produkt mit einer Säurezahl von 4 mg KOH/g isoliert.

## Beispiel 10

Man verfährt wie in Beispiel 8, setzt aber anstelle von Zinkstearat 2 g Zinkhydroxid ein. In diesem Fall ist die Säurezahl bereits nach 2 Stunden auf einen Wert von 4 mg KOH/g abgesunken. Nach einer weiteren Stunde wird die Reaktion bei einer Säurezahl von 2 mg KOH/g beendet.

## Patentansprüche

1. Verfahren zur Herstellung von Wachsestern aus gegebenenfalls teilverseiften Hart-paraffin-Oxidaten synthetischer Herkunft einerseits, und ein- oder mehrwertigen Alkoholen andererseits, in der Schmelze unter schonenden Bedingungen mit weitgehender bis vollständiger Veresterung der Oxidate,
dadurch gekennzeichnet,
daß man die Umsetzung in Gegenwart von 0,01 bis 3,0 Gew.-% Zinkverbindungen, bezogen auf die Einsatzmenge Hartparaffin-Oxidat, mit einem Alkoholzusatz stöchiometrisch zur halben bis doppelten Säurezahl, bei 140 bis 220°C, unter Abführung des Reaktionswassers als Dampf, bei mindestens 70 %-iger Veresterung der durch die Säurezahl erfaßten Wachssäuren und gleichzeitiger Senkung der Säurezahl des gebildeten Wachsesters unter 10 mg KOH/g, ausführt.

2. Verfahren nach Anspruch 1,
dadurch gekennzeichnet,
daß die Zinkverbindung ein Oxid, ein Hydroxid oder ein Mineralsäuresalz ist.

3. Verfahren nach Anspruch 1,
dadurch gekennzeichnet,
daß die Zinkverbindung das Salz einer Carbonsäure ist.

4. Verfahren nach den Ansprüchen 1 bis 3,
dadurch gekennzeichnet,
daß das Hartparaffin-Oxidat ein mittels Luft oxidiertes Produkt aus der Fischer-Tropsch-Synthese oder ein oxidiertes Produkt aus der Ethylenpolymerisation nach Ziegler ist.

5. Verfahren nach Anspruch 4,
dadurch gekennzeichnet,
daß das Hartparaffin-Oxidat eine Säurezahl von 5 bis 80 besitzt und in der Schmelze mittels Luft in Gegenwart eines Zinksalzes aus einem Produkt aus der Ethylenpolymerisation nach Ziegler mit einem Molekulargewicht (dampfdruckosmometrisch bestimmt) von 1 000 bis 4 000 erhalten wurde.

## Claims

1. A process for the preparation of a wax ester, made from an optionally partially-hydrolyzed hard paraffin oxidate of synthetic origin on the one hand and a monohydric or polyhydric alcohol on the other hand, in the melt under mild conditions with substantial to complete esterification of the oxidate, characterised in that the reaction is carried out at 140 to 220°C in the presence of 0.01 to 3.0 % by weight of zinc compound, relative to the amount of hard paraffin oxidate employed, with addition of half to twice the stoichiometric amount of alcohol relative to the acid number and removal of the water of reaction as steam, at least 70 % of the wax acid, determined by the acid number, being esterified and the acid number of the wax ester formed simultaneously being reduced to below 10 mg of KOH/g.

2. A process according to claim 1, characterised in that the zinc compound is an oxide, a hydroxide or a mineral acid salt.

3. A process according to claim 1, characterised in that the zinc compound is the salt of a carboxylic acid.

4. A process according to any of claims 1 to 3, characterised in that the hard paraffin oxidate is an air-oxidised product from the Fischer-Tropsch synthesis or an oxidised product from the Ziegler polymerisation of ethylene.

5. A process according to claim 4, characterised in that the hard paraffin oxidate has an acid number from 5 to 80 and has been obtained in the melt by means of air in the presence of a zinc salt from a product obtained from the Ziegler polymerisation of ethylene and having a molecular weight (determined by vapour pressure osmometry) from 1,000 to 4,000.

## Revendications

1. Procédé de préparation d'esters cireux à partir de produits d'oxydation de paraffine dure éventuellement partiellement saponifiés et de provenance synthétique, d'une part, et de mono- ou poly-alcools, d'autre part, à l'état fondu, dans des conditions ménageantes, avec une estérification large à complète des produits d'oxydation, caractérisé par le fait qu'on effectue la réaction en présence de 0,01 à 3,0 % en poids de composés du zinc, relativement à la quantité du produit d'oxydation de paraffine dure qui est utilisée, avec une addition d'alcool stoechiométrique par rapport à la moitié jusqu'au double de l'indice d'acide, à une température de 140 à 220°C, avec évacuation de l'eau réactionnelle à l'état de vapeur, pour une estérification d'au moins 70 % des acides cireux indiqués par l'indice d'acide et pour un abaissement simultané, au-dessous de 10 mg de KOH/g, de l'indice d'acide de l'ester cireux formé.

2. Procédé selon la revendication 1, caractérisé par le fait que le composé de zinc est un oxyde, un hydroxyde ou un sel d'acide minéral.

3. Procédé selon la revendication 1, caractérisé par le fait que le composé de zinc est le sel d'un acide carboxylique.

4. Procédé selon les revendications 1 à 3, caractérisé par le fait que le produit d'oxydation de paraffine dure est un produit provenant de la synthèse Fischer-Tropsch et oxydé avec de l'air, ou bien un produit oxydé provenant de la polymérisation de l'éthylène selon Ziegler.

5. Procédé selon la revendication 4, caractérisé par le fait que le produit d'oxydation de paraffine dure possède un indice d'acide de 5 à 80 et qu'il a été obtenu à l'état fondu avec de l'air en présence d'un sel de zinc, à partir d'un produit obtenu par polymérisation d'éthylène selon Ziegler et présentant un poids moléculaire (déterminé par voie osmométrique sous pression de vapeur) de 1 000 à 4 000.